# EUROPEAN PATENT SPECIFICATION

(11) **EP 1 579 859 B1**
(45) Date of publication and mention of the grant of the patent: **13.12.2006**
(21) Application number: 04007177.1
(22) Date of filing: 25.03.2004
(51) Int. Cl.: A61K 31/18, A61K 31/38, A61K 31/4035, A61P 25/00

(54) **Use of N-(2-aryl-propionyl)-sulfonamides for the treatment of spinal cord injury**
Verwendung von N-(2-Aryl-propionyl)-sulfonamiden zur Behandlung von Rückenmarkverletzungen
Utilisation de N-(2-aryl-propionyl)-sulfonamides dans le traitement des lésions de la moelle épinière

(43) Date of publication of application: 28.09.2005
(73) Proprietor: DOMPE' pha.r.ma S.p.A., 67100 L'Aquila (IT)
(72) Inventor: Bertini, Riccardo, 67100 L'Aquila (IT); Colotta, Francesco, 67100 L'Aquila (IT)
(74) Representative: Minoja, Fabrizio

(56) References cited:
- EP-B- 1 123 276
- WO-A-01/79189
- WO-A-02/062330
- US-A1- 2001 016 195
- LI J J: "Small molecule interleukin-8 modulators" EXPERT OPINION ON THERAPEUTIC PATENTS 2001 UNITED KINGDOM, vol. 11, no. 12, 2001, pages 1905-1910, XP001079136 ISSN: 1354-3776
- SCHNELL L ET AL: "Neutrophil-mediated axonal damage in the adult rat spinal cord" SOCIETY FOR NEUROSCIENCE ABSTRACTS, vol. 27, no. 2, 2001, page 1835, XP001199809 & 31ST ANNUAL MEETING OF THE SOCIETY FOR NEUROSCIENCE; SAN DIEGO, CALIFORNIA, USA; NOVEMBER 10-15, 2001 ISSN: 0190-5295
- XU J ET AL: "POLYMORPHONUCLEAR PMN CELL INFILTRATION IN EXPERIMENTAL SPINAL CORD INJURY" SOCIETY FOR NEUROSCIENCE ABSTRACTS, vol. 13, no. 3, 1987, page 1500, XP008034241 & 17TH ANNUAL MEETING OF THE SOCIETY FOR NEUROSCIENCE, NEW ORLEANS, LOUISIANA, USA, NOVEMBER 16-21, 19 ISSN: 0190-5295
- ISAKSSON JONAS ET AL: "Expression of ICAM-1 and CD11b after experimental spinal cord injury in rats" JOURNAL OF NEUROTRAUMA, vol. 16, no. 2, February 1999 (1999-02), pages 165-173, XP008034243 ISSN: 0897-7151
- CALVILLO LAURA ET AL: "Reduction of ischemia-reperfusion injury in the rat in vivo by DF1681A, an inhibitor of interleukin-8" JOURNAL OF THE AMERICAN COLLEGE OF CARDIOLOGY, vol. 37, no. 2 Supplement A, February 2001 (2001-02), page 374A, XP001199808 & 50TH ANNUAL SCIENTIFIC SESSION OF THE AMERICAN COLLEGE OF CARDIOLOGY; ORLANDO, FLORIDA, USA; MARCH 18-21, 2001 ISSN: 0735-1097
- KUNIHARA T ET AL: "Proinflammatory cytokines in cerebrospinal fluid in repair of thoracoabdominal aorta" ANNALS OF THORACIC SURGERY 2001 UNITED STATES, vol. 71, no. 3, 2001, pages 801-806, XP001199810 ISSN: 0003-4975

## Description

The present invention concerns the use of N-(2-aryl-propionyl)-sulfonamides of general formula (**I**): 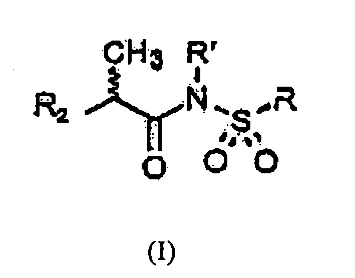 in which
R₂ is an aryl group,
R is a straight or branched C₁-C₆-alkyl, trifluoromethyl, cyclohexyl, o-tolyl, 3-pyridyl, 2-pyridyl-ethyl, p-cyano-phenylmethyl, p-aminophenylmethyl, 3-cyano-1-propyl, 4-aminobutyl group, an alkoxyethylene CH₃-(CH₂)ₙᵢ-(OCH₂CH₂)ₘᵢ- group in which nᵢ is zero or 1 and mᵢ is an integer from 1 to 3, or a P₁P₂N-CH₂-CH₂- group in which P₁ and P₂ are independently H, C₁-C₃- alkyl, benzyloxy-carbonyl, α- or *β*-pyridocarbonyl, carboxycarbonyl or carbalkoxycarbonyl, or P₁ and P₂ when joined to the N atom which they are linked to, form a phthalimido, piperidino, morpholino residue;
R' is H or straight or branched C₁-C₃-alkyl, preferably hydrogen, for the preparation of a medicament for the treatment of spinal cord injury.

### Background of the invention

Spinal cord injury (SCI) is one of the most frustrating conditions in neurology and medicine. The vast majority of SCI patients are young, and most survivors of significant injury face the prospects of limited recovery and permanent disability. The incidence of new SCI case is high, exceeding 12.000 new cases per year of paraplegia or quadriplegia in the United States (Sekhon L. et al. *Spine* 26, S2-S12, 2001). Yet with improved management, the mortality rate of SCI has steadily fallen. As a result, the prevalence of patients disabled by SCI now approximates 200.000 in the Unites States alone. The need for effective acute intervention, both to limit the numbers of permanently impaired patients and to give real hope to the newly injured, is particularly felt.

Current treatment of SCI is limited to high-dose glucocorticoid therapy, which is useful only when administered within hours of injury (Bracken M.B. et al. *The New England Journal of Medicine* 322, 1405-1411, 1990). The mechanisms by which the steroids exert their moderately beneficial effects remain unclear, though they are generally attributed to the protective effects on lipid peroxidation. Indeed, methylprednisolone suppresses the breakdown of membrane and neurofilament by inhibiting lipid peroxidation in injured spinal cord (Braughler J.M. et al. *J*. *Neurosurg* 67, 102-105, 1987). Yet, despite the fundamental inadequacy, high-dose glucocorticoid treatment has remained the only available therapy for SCI.

The pathogenesis of SCI is now known to involve cytokines, particularly Tumor Necrosis Factor (TNF), the expression of which contribute to neuronal death after SCI (Beattie M.S. et al. *Progress in Brain Research* 137, 37-47, 2002) and leukocytes infiltration. Indeed, SCI results in both primary injury, characterized by disruption of neural and vascular structure, and a cascade of secondary processes that collectively lead to additional loss of tissue. Post-traumatic inflammation, characterized by the accumulation of activated microglia and leukocytes, is thought to contribute to secondary pathogenesis (Mautes A.E.M. et al. *Physical Therapy* 80, 673-687, 2000). Strategies aimed at blocking neutrophil or macrophages influx and at inhibition of phagocytic and secretory activity of macrophages in the injured spinal cord have resulted in neuroprotection and improved locomotory function (Giulian D. et al. *Ann. Neurol.* 27, 33-42,1990; Taoka Y. et al. *Neuroscience* 79, 1177-1182, 1997).

Indeed, according to the available knowledge, the selective inhibition of interleukin-8 (CXCL8)- induced chemotaxis is not a sufficient condition for the protection of SCI. In fact, the scientific literature identified numerous factors involved in the etiology of the SCI, among which factors, CXCL8 does not certainly appear as one of the most important: for example Taoka Y. et al. *(Journal of Neurotrauma* 18, 533-543, 2001) report that leukocytopenia and inhibition of leukocyte recruitment by administration of an anti-P-selectin monoclonal antibody, an aspecific blocker of leukocyte adhesion, significantly reduced motor disturbances observed following SCI. In addition, recent research has shown elevated plasma levels of inflammatory mediators, including interleukin-2, interleukin-6, the soluble intereleukin-2 receptor, and intercellular adhesion molecule-1 (ICAM-1) in patients with long-standing SCI, as possible pathogenetic factors of the delay in the functional recovery (Segal J.L. et al. *Arch. Phys. Med. Rehabil.* 78, 44-47, 1997). It follows that, from the literature data, an aspecific inhibitor of the inflammatory response or, at least, of leukocyte recruitment would appear necessary for the inhibition of SCI.

The N-(2-aryl-propionyl)-sulfonamides of general formula (**I**) above are disclosed in EP 1123276 and in European Patent Application EP 04101202.2. The sulfonamides described therein are reported to be useful, for example, in the prevention and treatment of tissue damage due to exacerbated recruitment of polymorphonuclear neutrophils (PMN leukocytes) at the inflammatory sites.

### Description of the invention

It has now surprisingly been found that said sulfonamides of formula (**I**), and particularly the sulfonamides of formula (**Ia**) 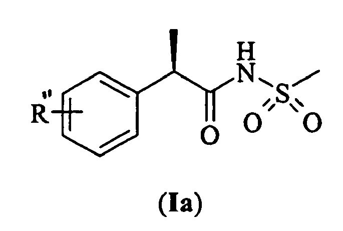 wherein R" represents one to three substituents, which are the same or different, selected from hydrogen, halogen atoms, C₁-C₄-alkyl, C₁-C₄-alkoxy, hydroxy, C₁-C₇-acyloxy, cyano, nitro, amino, C₁-C₃-acylamino, halo C₁-C₃-alkyl, halo C₁-C₃-alkoxy, benzoyl, 4-(2-methyl-propyl)-phenyl, 3-phenoxy-phenyl, 2-[4-(1-oxo-2-isoindolinyl)phenyl], 5-benzoyl-thien-2-yl, 4-thienoyl-phenyl, C₁-C₂-halogenoalkylsulphonyloxy, are effective in the protection from functional injury of SCI.

R", in the compounds of formula (**Ia**), preferably represents hydrogen, 4-isobutyl, 3-benzoyl, **4**-trifluoromethanesulphonyloxy.

The protection from functional injury of SCI has been demonstrated in an experimental model in rats, disclosed in detail hereinafter, using two representative compounds of formula (**I**), namely the compound of formula (**II**) and its lysine salt (L-lysine or DL-lysine), and the compound of formula (**III**). Both compounds were found very active in this in vivo model.

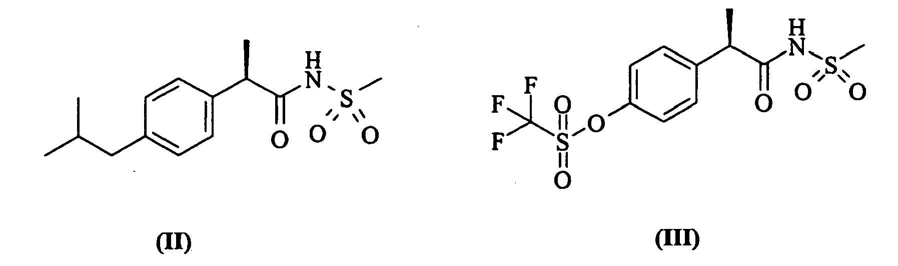

Compound of formula **(II)** and compound of formula **(III)** also reduced tissue injury, evaluated as extension of post-traumatic cavity, oligodendrocytes apoptosis and leukocyte infiltration.

The invention is illustrated in the following Example.

### EXAMPLE

Adult Sprague-Dawley rats (females) weighing 240-260 g were maintained in the animal facilities under standard housing conditions (22 ± 2°C, 65% humidity, artificial light from 06.00-20.00 h). A standard dry diet and water were available *ad libitum.*

SCI in the rat was performed as previously reported (Gorio A. et al. *Proc. Natl. Acad. Sci. USA* 99, 9450-9455, 2002). The lesioning apparatus is computer controlled and free of the influence of gravity force. The force applied was 1N per 1 second.

Recovery from hind limb disability was evaluated by means of "free locomotion test" performed 24 hours, 4, 7, 11, 15, 19 and 27 days after SCI.

The "free locomotion test" allows the detection of feet positioning, and joint rotation. The quality of functional recovery is quantitatively expressed according to the "BBB scale" developed at the Ohio University. Such a test allows the quantification of rat hind limb free locomotion deficits by observing their movements in an open space free of obstacles:
0- Lesioned rat cannot move either limbs
1- Small movement of a joint (heap or knee)
From 2 to 6- Movement in progressive extension of the 3 joints
7- Good movement of the 3 joints
8- Animals walk without plantar support of the weight
From 9 to 11- Animals walk from occasionally to progressively frequent with plantar support of the weight.
12- Occasional coordination of hind limbs and forelimbs during walk
From 13 to 14- Progressive coordination with the forelimbs.
15- Consistent plantar support of weight and coordination during walk; occasional movement of fingers during advancement.
From 16 to 18- Progressive tendency to finger movements; during walk the foot is predominantly in parallel position to the body.
19- The foot position is correctly parallel to the body, and the tail is maintained low during walk.
20- Wobbling lateral and unstable locomotion
21- Normal condition

Apoptosis of oligodendrocytes was determined at the level of the gracilis and cuneatus fascicle (3 mm rostrally from the site of contusion injury) 28 days after SCI using the terminal deoxynucleotidyltransferase-mediated dUTP and labeling (TUNEL) methodology.

Leukocyte infiltration was quantitatively estimated by CD68 positive cells 1 and 7 days after SCI.

Extension of post-traumatic cavity was performed by classical histological techniques 28 days after SCI.

The following experimental groups of animals were considered:
**Group 1** (n=28) rats treated with saline solution after SCI
**Group 2** (n=28) rats treated with compound of formula **(II)** after SCI
**Group 3** (n=28) rats treated with compound of formula **(III)** after SCI

Animals were treated with saline or compound of formula **(II)** (15 mg/kg) by i.v. injection within 30 minutes after SCI, then s.c. every 2 hours in the following 6 hours. The following days the animals were treated s.c. at 8 am and 5 pm until the 7^{th} day after SCI. Animals were treated with compound of formula **(III)** (8 mg/kg) by i.v. injection within 30 minutes after SCI, then s.c. 24 hours after SCI. The following days the animals were treated s.c. every 36 hours until the 7^{th} day after SCI.

Data were analyzed by ANOVA followed by Dunnett's *t* test. Statistical significance was accepted at *P*<0.05.

### Results

The effect of compound of formula **(II)** and compound of formula **(III)** on functional recovery (motor score), quantitatively expressed according to the "BBB scale", was evaluated at different times after SCI. Figure (1) shows the effect of (**R**)-ibuprofen methanesulfonamide, and figure (2) shows the effect of R-2-[(4'-trifluoromethanesulphonyloxy)phenyl]-N-methanesulfonyl propionamide. All animals subjected to SCI were profoundly affected immediately after injury (motor score 0 for all groups) and significant recovery was not evident in vehicle (saline) treated group until the 7^{th} day after SCI. Treatment with compound of formula **(II)** and compound of formula **(III)** significantly promoted functional hind limb recovery after SCI. The recovery was progressive, being the most effective period between the 4^{th} and the 11^{th} day after SCI.

Immunohistologic evaluation of leukocyte infiltration was evaluated 1 day and 7 days after SCI. As shown in Table 1, compound of formula **(II)** dramatically reduced leukocyte infiltration (80% of inhibition) at 24 hours and 7 days after SCI. A similar inhibition of leukocyte recruitment was also observed in rats treated with compound of formula **(III)** (data not shown).

It is well known that apoptosis of oligodendrocytes is a crucial event during the early stages after traumatic lesion of the spinal cord, and that the extend of neurological recovery is also dependent on how such process can be counteracted or attenuated. Oligodendrocyte death causes demyelination of the axons spared by the lesion, thus causing loss of the ability to conduct the electrical impulse across the lesion site. The pharmacological attenuation of oligodendrocyte apoptosis is thus a primary target of any pharmacological treatment aiming at promoting recovery after SCI. As shown in Table 2, treatment with compound of formula (**II**) and compound of formula (**III**) blocked oligodendrocyte apoptosis determined 28 days after SCI [85% and 65% of inhibition after treatment of rats with (R)-ibuprofen methanesulfonamide and R-2-[(4'-trifluoromethanesulphonyloxy)phenyl]-N-methanesulfonyl propionamide, respectively].

Finally, it was investigated the effect of compound of formula (**II**) and compound of formula (**III**) on tissue damage induced by SCI. As shown in Table 3, treatment with compounds described above significantly reduced tissue damage at the site of the lesion and the extension of post-traumatic cavity 28 days after SCI.

In conclusion, data reported above clearly show how compound of formula (**II**) and compound of formula (**III**) can be advantageously used in medical practice in the promotion of functional recovery after SCI.

**Table 1. Number of infiltrated leukocytes (mean ± SE; n=8)**

| **Time from SCI** | **1 day** | | **7 days** | |
|---|---|---|---|---|
| | Saline | Formula **(II)** | Saline | Formula **(II)** |
| Epicenter | 125±36 | 24±3*** | 235±54 | 19±3*** |
| Periphery | 56±13 | 3±1*** | 99±32 | 4±2*** |

| | | | | |
|---|---|---|---|---|
| ***P<0.001 (R)-ibuprofen methanesulfonamide treated animals vs saline treated animals | | | | |

**Table 2. Number of oligodendrocyte apoptotic nuclei (mean ± SE; n=12)**

| **Treatment** | |
|---|---|
| Saline | 14.9±2 |
| (R)-ibuprofen methanesulfonamide | 2.1±1.2* |
| R-2-[(4'-trifluoromethanesulphonyloxy)phenyl]-N-methanesulfonyl propionamide | 5 .2±3.8* |

| | |
|---|---|
| *P<0.05 and ***P<0.001 drug treated animals vs saline treated animals | |

**Table 3. Percentage of spared tissue at lesion site (mean ± SE; n=12)**

| **Treatment** | Lesion Epicenter | Cavity Volume |
|---|---|---|
| Saline | 39.8±3.9 | 46.6±3.1 |
| (R)-ibuprofen methanesulfonamide | 48.9±3.0** | 58.2±2.9** |
| R-2-[(4'-trifluoromethanesulphonyloxy)phenyl]-N-methanesulfonyl propionamide | 49.7±4.2** | 60.4±4.3** |

| | | |
|---|---|---|
| **P<0.01 drug treated animals vs saline treated animals | | |

For the considered therapeutical purposes, suitable pharmaceutical compositions may be prepared using conventional techniques and excipients such as those described in "Remington's Pharmaceutical Sciences Handbook" Mack Publishing Co., New York, 18^{th} Ed., 1990.

The compositions of the invention will preferably be administered intramuscularly, intravenously, as bolus, in view of the urgency character of the pathology to be treated, even though other administration routes cannot be excluded, for instance the oral route.

The average daily dosage will depend on various factors such as severity of the disease and conditions of the patient (age, sex and weight). The dose will generally vary from 1 or a few mg to 1500 mg of the compounds daily, optionally subdivided in multiple administrations. Higher dosages can also be administered thanks to the low toxicity of the compounds of the invention, even for long-term treatments.

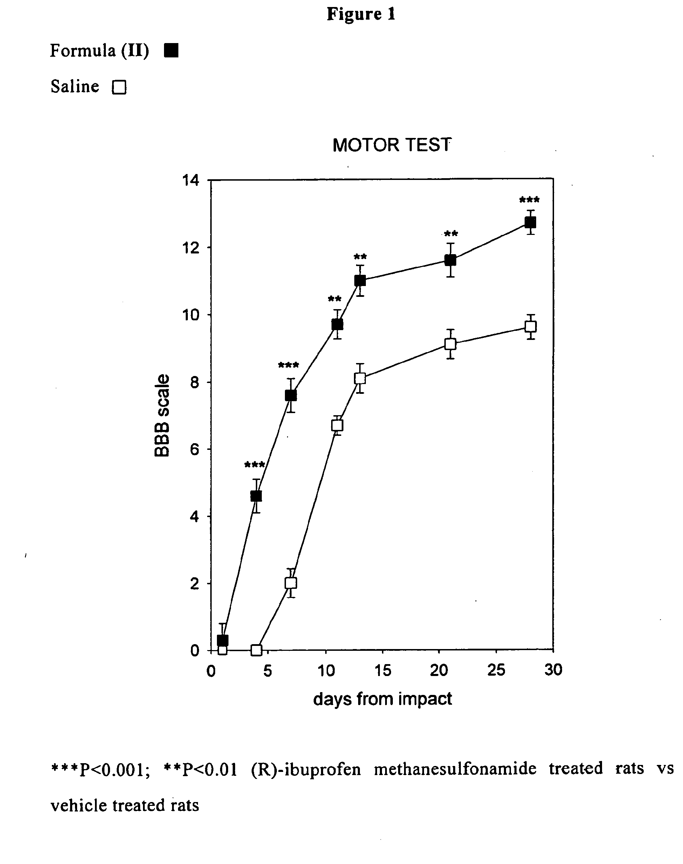

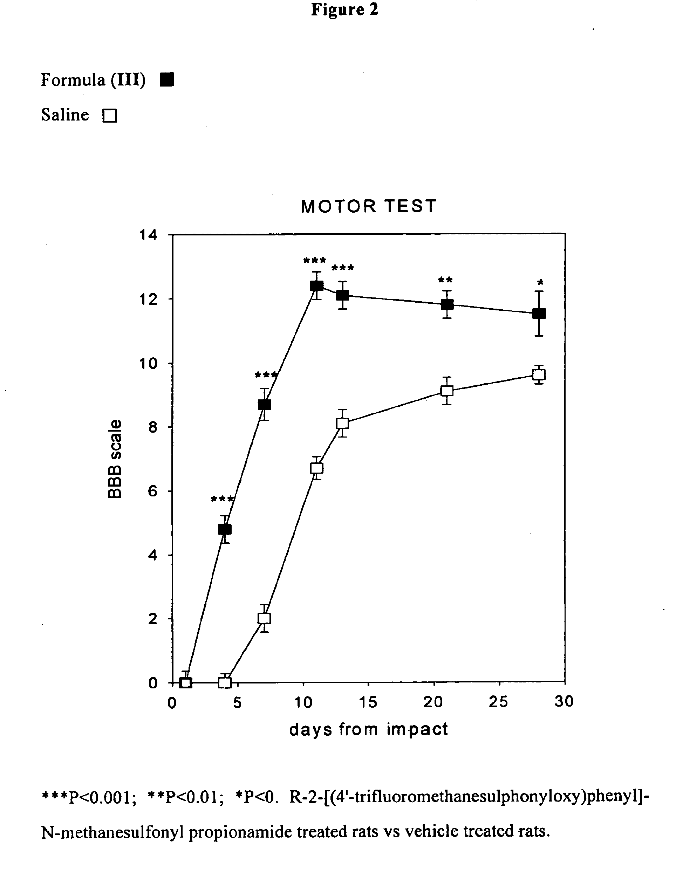

## Claims

1. Use of N-(2-aryl-propionyl)-sulfonamides of general formula (I): 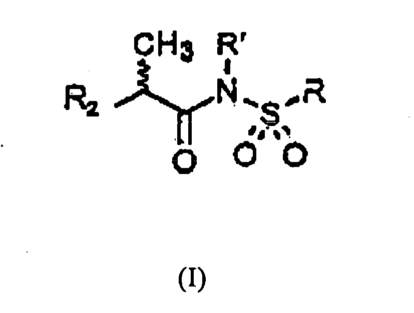 in which
R₂ is an aryl group,
R is a straight or branched C₁-C₆-alkyl, trifluoromethyl, cyclohexyl, o-tolyl, 3-pyridyl, 2-pyridyl-ethyl, p-cyano-phenylmethyl, p-aminophenylmethyl, 3-cyano-1-propyl, 4-aminobutyl group, an alkoxyethylene CH₃-(CH₂)ₙᵢ-(OCH₂CH₂)ₘᵢ- group in which nᵢ is zero or 1 and mᵢ is an integer from 1 to 3, or a P₁P₂N-CH₂-CH₂- group in which P₁ and P₂ are independently H, C₁-C₃- alkyl, benzyloxy-carbonyl, α-, or β- pyridocarbonyl, carboxycarbonyl or carbalkoxycarbonyl, or P₁ and P₂, when joined to the N atom which they are linked to, form a phthalimido, piperidino, morpholino residue;
R' is H or straight or branched C₁-C₃-alkyl, preferably hydrogen, for the preparation of a medicament for the treatment of spinal cord injury.

2. Use according to claim 1 of the compounds of formula **(Ia)** 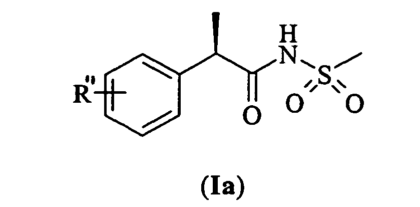 wherein R" represents one to three substituents, which are the same or different, selected from hydrogen, halogen atoms, C₁-C₄-alkyl, C₁-C₄-alkoxy, hydroxy, C₁-C₇-acyloxy, cyano, nitro, amino, C₁-C₃-acylamino, halo C₁-C₃-alkyl, halo C₁-C₃-alkoxy, benzoyl, 4-(2-methyl-propyl)-phenyl, 3-phenoxy-phenyl, 2-[4-(1-oxo-2-isoindolinyl)phenyl], 5-benzoyl-thien-2-yl, 4-thienoyl-phenyl, C₁-C₂-halogenoalkylsulphonyloxy.

3. Use according to claim 2 wherein R" represents hydrogen, 4-isobutyl, 3-benzoyl, 4-trifluoromethanesulphonyloxy.

4. Use according to claim 3 of the compounds of formula (II) and (III).

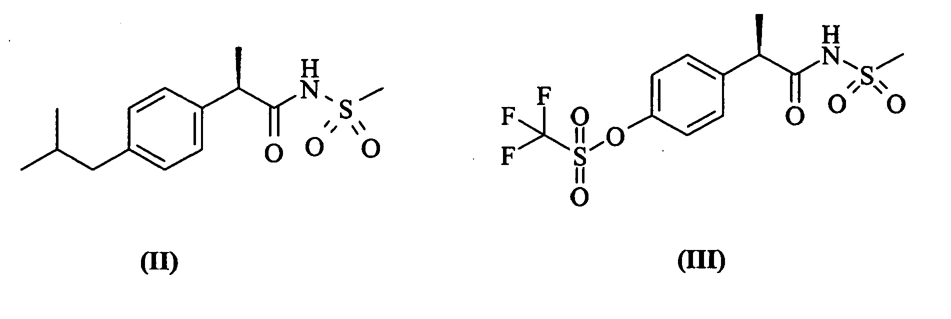

## Patentansprüche

1. Verwendung von N-(2-Aryl-propionyl)-sulfonamiden der allgemeinen Formel (I): 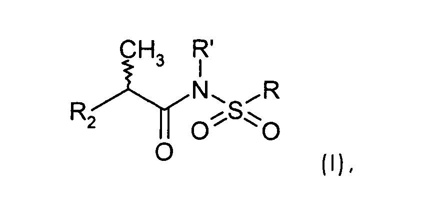 in welcher
R² eine Arylgruppe ist,
R eine geradkettige oder verzweigte C₁ - C₆-Alkyl-, Trifluormethyl-, Cyclohexyl-, o-Tolyl-, 3-Pyridyl-, 2-Pyridylethyl-, p-Cyanophenylmethyl-, p-Aminophenylmethyl-, 3-Cyano-1-propyl-, 4-Aminobutylgruppe, eine Alkoxyethylen CH₃-(CH₂)ₙᵢ-(OCH₂CH₂)ₘᵢ-Gruppe, in welcher nᵢ 0 oder 1 ist und mᵢ eine ganzzahlige Zahl von 1 bis 3 ist, oder eine P₁P₂N-CH₂-CH₂-Gruppe ist, in welcher P₁ und P₂ unabhängig voneinander H, C₁ - C₃-Alkyl, Benzyloxycarbonyl, α- oder β-Pyridocarbonyl, Carboxycarbonyl oder Carbalkoxycarbonyl sind oder P₁ und P₂ zusammen mit dem N-Atom, an welches sie gebunden sind, einen Phthalimido-, Piperidino-, Morpholinorest bilden;
R' H oder geradkettiges oder verzweigtes C₁ - C₃-Alkyl, vorzugsweise Wasserstoff, ist,
zur Herstellung eines Arzneimittels zur Behandlung von Rückenmarkverletzungen.

2. Verwendung nach Anspruch 1 der Verbindungen der Formel (Ia) 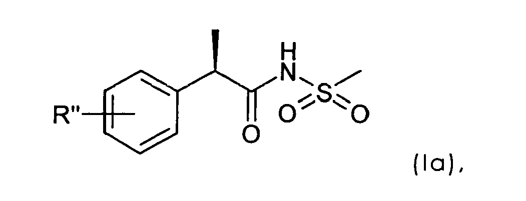 in welcher R" einen bis drei Substituenten darstellt, welche gleich oder verschieden sind, ausgewählt aus Wasserstoff, Halogenatomen, C₁ - C₄-Alkyl, C₁ - C₄-Alkoxy, Hydroxy, C₁ - C₇-Acyloxy, Cyano, Nitro, Amino, C₁ - C₃-Acylamino, Halo-C₁-C₃-alkyl, Halo-C₁-C₃-alkoxy, Benzoyl, 4-(2-Methylpropyl)-phenyl, 3-Phenoxyphenyl, 2-[4-(1-Oxo-2-isoindolinyl)phenyl], 5-Benzoyl-thien-2-yl, 4-Thienoylphenyl, C₁ - C₂-Halogenalkylsulfonyloxy.

3. Verwendung nach Anspruch 2, worin R" Wasserstoff, 4-Isobutyl, 3-Benzoyl, 4-Trifluormethansulfonyloxy darstellt.

4. Verwendung nach Anspruch 3 der Verbindungen der Formel (II) und (III)

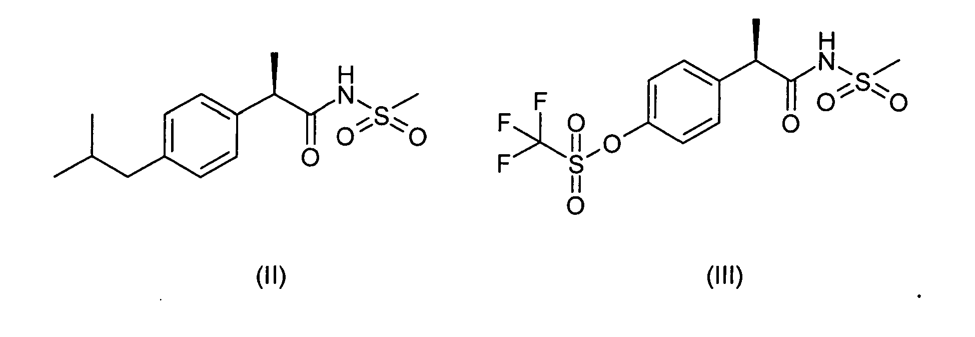

## Revendications

1. Utilisation de N-(2-arylpropionyl)sulfonamides de formule générale (I) : 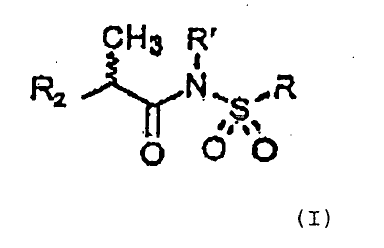 dans laquelle
R₂ est un groupe aryle,
R est un groupe alkyle en C₁-C₆ linéaire ou ramifié, trifluorométhyle, cyclohexyle, o-tolyle, 3-pyridyle, 2-pyridyléthyle, p-cyanophénylméthyle, p-aminophénylméthyle, 3-cyano-1-propyle, 4-aminobutyle, un groupe alkoxyéthylène CH₃-(CH₂)ₙᵢ-(OCH₂CH₂)ₘᵢ- dans lequel ni est zéro ou 1 et mi est un entier de 1 à 3, ou un groupe P₁P₂N-CH₂-CH₂- dans lequel P₁ et P₂ sont indépendamment H, alkyle en C₁-C₃, benzyloxycarbonyle, α- ou β-pyridocarbonyle, carboxycarbonyle ou carbalkoxycarbonyle, ou P₁ et P₂, quand ils sont unis à l'atome d'N auquel ils sont liés, forment un résidu phtalimido, pipéridino, morpholino ;
R' est H ou alkyle en C₁-C₃ linéaire ou ramifié, de préférence hydrogène,
pour la préparation d'un médicament pour le traitement d'une lésion de la moelle épinière.

2. Utilisation selon la revendication 1 des composés de formule (Ia) 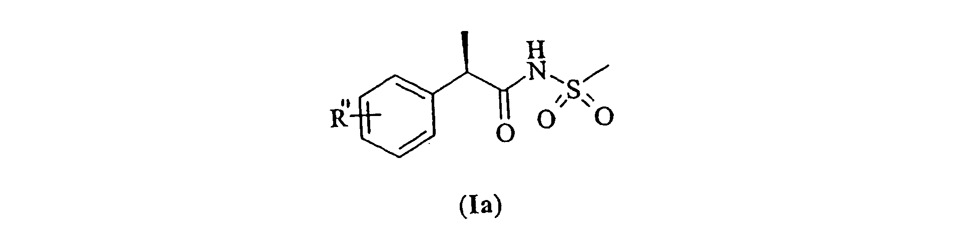 dans laquelle R'' représente un à trois substituants, qui sont identiques ou différents, choisis parmi hydrogène, des atomes d'halogène, alkyle en C₁-C₄, alkoxy en C₁-C₄, hydroxy, acyloxy en C₁-C₇, cyano, nitro, amino, acylamino en C₁-C₃, halo-alkyle en C₁-C₃, halo-alkoxy en C₁-C₃, benzoyle, 4-(2-méthylpropyl)phényle, 3-phénoxy-phényle, 2-[4-(1-oxo-2-isoindolinyl)phényle], 5-benzoyl-thién-2-yle, 4-thiénophényle, (halogénoalkyl en C₁-C₂)sulfonyloxy.

3. Utilisation selon la revendication 2 pour laquelle R'' représente hydrogène, 4-isobutyle, 3-benzoyle, 4-trifluorométhanesulfonyloxy.

4. Utilisation selon la revendication 3 des composés de formules (II) et (III).
